# EUROPEAN PATENT SPECIFICATION

(11) **EP 2 024 423 B1**
(45) Date of publication and mention of the grant of the patent: **09.12.2009**
(21) Application number: 07733689.9
(22) Date of filing: 16.05.2007
(51) Int. Cl.: C08G 77/388, A61K 8/898

(54) **AMINO-ACID FUNCTIONAL SILOXANES, METHODS OF PREPARATION AND APPLICATIONS**
AMINOSÄUREFUNKTIONELLE SILOXANE, HERSTELLUNGSVERFAHREN UND ANWENDUNGEN
SILOXANES PORTANT UNE FONCTION AMINOACIDE, MÉTHODES DE SYNTHÈSE ET APPLICATIONS

(30) Priority: 08.06.2006 GB 0611217
(43) Date of publication of application: 18.02.2009
(73) Proprietor: Dow Corning Corporation, Midland, Michigan 48611 (US)
(72) Inventor: DRAKE, ROBERT, CF64 5BR (GB); POSTIAUX, STEPHANIE, 7070 Le Roeulx (BE)
(74) Representative: Donlan, Andrew Michael
(86) International application number: PCT/GB2007/050268
(87) International publication number: WO 2007/141565

(56) References cited:
- US-B1- 6 358 501
- US-B2- 6 451 905

## Description

The present invention relates to a method for the production of amino-acid functional siloxanes, the products of the process and uses thereof including for example their use in personal care compositions.

US 5679819 describes a copolymer comprising units made up of cysteine, or a derivative thereof, bonded to a silicon-containing component, wherein the silicon-containing component contains at least one siloxane link". The method of preparation used is based on epoxy or anhydride functional siloxanes. EP 1149855 describes an alternative method of preparation of amino acid functional siloxanes using anhydride functional siloxane starting materials. WO 00/49090 discloses shear stable amino-silicone emulsions, prepared by the addition of monoacids to the amino-siloxane in aqueous suspension and reaction to give the salt. JP A Sho 50-158700 describes the preparation of functional siloxanes via a method involving the amino acids containing N-acylamino groups which are reacted with siloxanes having alkyl halide groups, however this method has the disadvantage that because the carboxy groups of the amino acids are involved in the reaction, the carboxy groups derived from the amino acids are absent from the resulting functional organopolysiloxanes, which thus lack the properties normally associated with amino acids. JP2004-182680 describes an oily cosmetic product containing a silicone polymer which has been modified with an amino acid derivative.

US 5272241 and US 5516869 describe a method of preparing an amino acid functional siloxane using the following process:-
I. hydrosilylating a silicone hydride with a lactam to form an amide functional siloxane; and
II. hydrolyzing the amide functional siloxane of (i) thereby preparing an amino acid functional siloxane.

In accordance with the present invention there is provided a method of preparing an amino acid functional siloxane by reacting an amino acid derivative selected from the group of an N-acyl amino acid and an N-aroyl amino acid with an amino functional siloxane optionally in the presence of a solvent.

The amino acid derivative is selected from N-acyl amino acids and/or N-aroyl amino acids. Any suitable N-acyl amino acid may be utilised in the process of the present invention. Preferably, the acyl group comprises from 1 to 10 carbon atoms and may be linear or branched, examples of suitable acyl groups include N-acetylated, N-propanoyl, N-butanoyl, N-pentanoyl and N-hexanoyl groups. The amino acid derivatives utilised in the present invention are preferably N-acetylated derivatives of naturally occurring amino acids but non-naturally occurring compounds containing the same functionality may be utilised and for the sake of this invention are included in the definition of amino acids. Examples include but are not restricted to N-acetylated amino acids in which no additional functionality is present on the amino acid side chain such as N-Acetyl-Glycine, N-Acetyl-Alanine, N-acetyltryptofan and N-Acetyl-Valine but may also include N-acetylated amino acids containing further functionality in the amino acid side chain such as N-Acetyl-Glutamine (1) or 2-Pyrrolidone-5-Carboxylic Acid (2) (a dehydrated form of Glutamic acid) 4-Acetamido-benzoic acid (3) and N-acetyltryptofan (4). 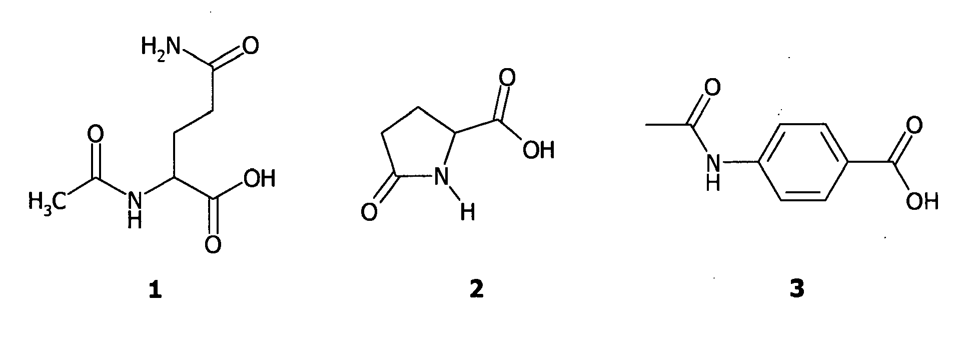 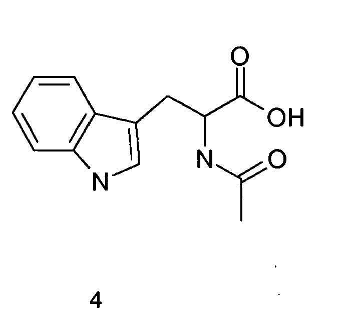 Whilst 4-Acetamido-benzoic acid (3) is not a naturally occurring a-amino acid, it does contain the same functionality as naturally occurring amino acids.

Any suitable N-aroyl amino acid may be utilised in the process of the present invention. The N-aroyl amino acid derivatives utilised in the present invention are preferably N-benzoyl derivatives or substituted N-benzoyl derivatives thereof.

Any suitable amino siloxane may be utilised in the method according to the present invention. The siloxane may comprise amino terminal groups or amine groups in the siloxane side chain. Preferably each amino group is bonded to a silicon atom via an alkyl group, i.e. is in the form of an alkyl amine. Each amine group may be a primary, secondary or tertiary amine group, preferably each amine group is primary or secondary but most preferably each amine group is a primary amine.

Preferably the aminosiloxane includes siloxane units of formula (5)

-(RₐSiO_{(4-a)/2})- (5)

in which each R is independently an organic group such as an aliphatic carbon group having from 1 to 10 carbon atoms optionally substituted with one or more amine, alkoxy, hydroxy, or fluorine and a is 0, 1 or 2. Particular examples of groups R include methyl, ethyl, propyl, butyl, vinyl, cyclohexyl, phenyl, tolyl group, aminoalkyl groups such as aminomethyl, aminoethyl, aminopropyl, aminobutyl, aminoisobutyl, aminocyclohexyl, aminophenyl or aminotolyl group, or an aminoalkylaminoalkyl group such as an aminoethylaminoisobutyl group (H₂NCH₂CH₂NHCH₂CH(CH₂) CH₂-) or an aminoethylaminopropyl group (H₂NCH₂CH₂NHCH₂CH₂CH₂-), an alkyl group substituted with fluorine such as 3,3,3-trifluoropropyl or beta-(perfluorobutyl)ethyl group. Suitably, at least some and preferably substantially all of the R groups are methyl or aminoalkyl groups, most preferably aminopropyl, aminoethyl, aminoisobutyl aminoethylaminopropyl and/or aminoethylaminoisobutyl group.

Preferably the aminosiloxane is a linear organopolysiloxane molecular chain (i.e. a = 2) for all chain units although some branching may occur. Preferred materials have polydiorganosiloxane chains according to the general formula (6)

-(R₂SiO)ₜ- (6)

in which each R is as defined above and is preferably a methyl group or an aminoalkyl group, preferably aminopropyl, aminoalkyl groups, most preferably aminopropyl, aminoethyl, aminoisobutyl aminoethylaminopropyl and/or aminoethylaminoisobutyl group, and t has a value of from 5 to 1000. Suitable polymers have viscosities of up to 1000 000 mPa.s at 25°C.

Preferred polysiloxanes containing units of formula (5) are thus polydiorganosiloxanes having terminal, silicon-bound aminoalkyl groups and/or terminal, silicon-bound alkyl radicals and/or terminal, silicon-bound alkoxy radicals. Preferably when the amino groups are present in the form of amino alkyl substituents on the polymer chain the polymer is terminated with trialkylsilyl groups or dialkylalkoxysilyl groups. The polydiorganosiloxanes may be homopolymers or copolymers. Mixtures of different polydiorganosiloxanes as described above are also suitable.

Preferably in the absence of a solvent the reaction is carried out at a temperature above room temperature (25°C) but below the boiling/decomposition point of the amino acid, typically at a temperature greater than 50°C and most preferably at a temperature between 75°C and 150°C (assuming 150°C is lower than the boiling/decomposition point of the amino acid concerned.

Preferably, in the absence of a solvent, the reaction is carried out in an inert atmosphere (e.g. nitrogen) or under vacuum.

The presence of a solvent in the method in accordance with the present invention is optional. Any suitable solvent may be utilised, examples include aromatic hydrocarbons such as benzene, toluene and xylene, aliphatic hydrocarbons such as hexane and pentane, ethers, alcohols, ketones such as acetone. Particularly preferred are alcohols such as ethanol, propanol, isopropanol, butanol, pentanol and hexanol or mixtures thereof. When a solvent is present the reaction may be carried out at a temperature above room temperature (25°C) but below the boiling/decomposition point of the amino acid and/or solvent concerned e.g. at a temperature of up to 100°C. However, more preferably in the presence of a solvent the reaction is carried at a temperature between room temperature and 50°C and most preferably at room temperature. When present the solvent preferably comprises at least 15% by weight of the total reaction mixture.

The applicants found that two alternative products were obtainable in the absence of a solvent, the salt and the amide as shown in the following reaction scheme in which PDMS stands for polydimethylsiloxane

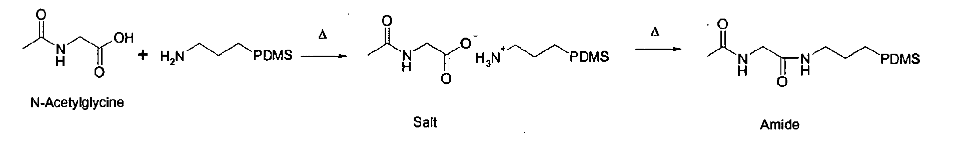

However, typically only the salt resulted in the presence of a solvent (typically an alcohol) as depicted in the following reaction scheme in which the solvent shown is ethanol (EtOH).

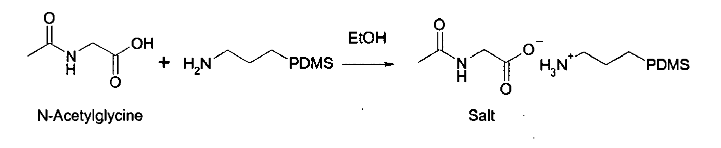

The inventors found that both the salt and the amine products of amino functional siloxanes and N-Acyl or N-aroyl amino acids particularly N-acetyl amino acids could be prepared thermally by mixing the reagents in the absence of solvent and heating under nitrogen or vacuum. In general it was found that the product obtained was at least partially dependent on the temperature of the reaction. Preferably in the absence of a solvent, allowing the reaction to proceed at a temperature of between 80 and 110°C, preferably about 100°C the resulting product was the salt, whilst when the reaction proceeded at a significantly higher temperature e.g. 140°C or greater the amide is formed. When seeking to prepare the amide the inventors considered that enabling the reaction to proceed under vacuum was preferred (although not essential)

The inventors found that products were easily prepared when reacting amino functional siloxanes with N-acyl amino acids providing that the side chain of the amino acid fragment was either hydrogen e.g. N-Acetyl-Glycine or an alkyl group e.g. N-Acetyl-Alanine and N-Acetyl-Valine. However, whilst successful the reaction taking place when the amino acid side chain contained additional functionality e.g. N-Acetyl-Glutamine (1) or 2-Pyrrolidone-5-Carboxylic Acid (2) (the dehydrated form of Glutamic acid) and 4-Acetamido-benzoic acid (3) were more difficult to prepare. 4-Acetamido-benzoic acid is not a naturally occurring a-amino acid but does contain the same functionality as the protected amino acids.

The inventors found that in addition to the thermal method discussed above, that the salts of N-acyl or N-aroyl amino acids with the amino siloxanes could be prepared much more simply by mixing the reagents in a solvent, particularly ethanol, as 15 to 70% w/w, most preferably about 50% w/w, and stirring at ambient temperature for 10 to 30 minutes. This then generally gave a clear viscous solution that could be dried down to give the desired product or further diluted as required.

In general it was found that for materials produced by both the thermal and the ethanol based routes that the observed properties were very similar. In the presence of a solvent (ethanol) no amide formation is observed and this may account for some minor differences from the thermal route where a few percent of the amide is generally present. The materials prepared by the "solvent" route generally display better clarity which, it is suggested, may be due to the absence of amide or better dispersion of the amino acid during synthesis.

Reactions between a variety of aminosiloxanes with non-N-acyl and non-N-aroyl amino acids such, aspartic acid, valine and glycyl-glycine failed to produce equivalent products.

In accordance with one embodiment of the present invention there is provided a siloxane salt compound obtainable by reacting an amino acid derivative selected from the group of an N-acyl amino acid and an N-aroyl amino acid with an amino functional siloxane optionally in the presence of a solvent.

In accordance with the present invention there is provided an amide compound obtainable method of preparing an amino acid functional siloxane by reacting an amino acid derivative selected from the group of an N-acyl amino acid and an N-aroyl amino acid with an amino functional siloxane optionally in the absence of a solvent.

The products in accordance with the present invention may be incorporated into compositions for topical applications in the form of suitable ointments, creams, gels, pastes, foams and/or aerosols or the like. Such compositions comprise at least the product of the present invention and an acceptable carrier material. The compositions for topical application may comprise the product of the present invention in the "oil" phase of either a water in oil emulsion or an oil in water emulsion composition which comprises an oil (nonaqueous) phase, an aqueous phase and incorporates an emulsifier. Typically the oil phase in such an emulsion is silicone based. However, where appropriate the product of the present invention may be introduced in the water phase of any such emulsion. It is to be appreciated however that the product of the present invention may be introduced into compositions for topical applications in any other suitable form such as for example shampoos, shower gels, rinse off conditioners.

Such compositions preferably are or form part of a personal care product. Such as for example antiperspirants; deodorants; skin creams; skin care lotions; moisturizers; facial treatments such as wrinkle control or diminishment treatments; exfoliates; body and facial cleansers; bath oils; perfumes; colognes; sachets; sunscreens; pre-shave and after-shave lotions; shaving soaps; shaving lathers; hair shampoos; hair conditioners; hair colorants; hair relaxants; hair sprays; mousses; gels; permanents; depilatories and cuticle coats; make-ups; colour cosmetics; foundations; concealers; blushes; lipsticks; eyeliners; mascaras; oil removers; colour cosmetic removers and powders; and medicament creams, pastes or sprays including anti-acne, dental hygienic, antibiotic, healing promotive, nutritive medicaments, and the like, and which may be preventative and/or therapeutic medicaments.

In one embodiment of the present invention the personal care product comprising the personal care composition as described above is a conditioning shampoo, a liquid hair gel or a conditioner and wherein the conditioner may be a rinse-off conditioner or a leave-in conditioner or the like for the treatment of hair providing one or more benefits of enhanced conditioning such as the provision of anti-static, anti-frizz, lubricity, shine, strengthening, viscosity, tactile, wet combing, dry combing, colour retention, heat protection, styling, or curl retention. In the case of a hair care product the product is administered using an efficacious amount of the personal care product.

In a further embodiment of the present invention there is provided a method of treating hair comprising: (1) administering a safe and effective amount of the personal care composition as recited in claim 1 to hair in need of treatment; and (2) distributing the personal care composition throughout the hair in need of treatment.

In an alternative embodiment the personal care product may be an antiperspirant provided in the form of a soft solid or a gel.

In a still further embodiment of the present invention the personal care product is used to treat the skin such as a cosmetic product and preferably provides an enhanced conditioning benefit. The compositions according to this invention can be used on the skin of humans or animals for example to moisturize, colour or generally improve the appearance or to apply actives, such as sunscreens, deodorants, insect repellents etc.

In a further embodiment of the present invention there is provided a method of treating skin comprising: (1) administering an efficacious amount of the personal care composition and (2) rubbing the personal care composition into the skin.

Preferably the product of the process of the present invention may be used in the manufacture of medicaments for suitable therapeutic applications.

The compositions according to this invention can be used by the standard methods, such as applying them to the human or animal body, e.g. skin or hair, using applicators, brushes, applying by hand, pouring them and/or possibly rubbing or massaging the composition onto or into the body. Removal methods, for example for colour cosmetics are also well known standard methods, including washing, wiping, peeling and the like.

The siloxane copolymers of the invention have particular use as conditioning agents for hair, making wet hair easier to comb and dry hair softer and easier to comb without imparting greasy or heavy characteristics to the hair. The siloxane copolymers have particular advantage in clear aqueous conditioners, forming conditioner compositions of improved clarity and maintaining that clarity for longer compared to compositions containing siloxane copolymers in which the relatively long alkyl groups are present as terminal groups on amidoalkyl groups or (polyoxyalkylene)-alkyl groups.

The use of compositions comprising the siloxanes according to the invention on hair may use a conventional manner for conditioning hair. An effective amount of the composition for conditioning hair is applied to the hair. Such effective amounts generally range from about 1g to about 50g, preferably from about 1g to about 20g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit. When a high silicone content is incorporated in a hair care composition according to the invention, this may be a useful material for split end hair products.

The siloxanes of the present invention may also be utilised in skin care applications such as cosmetics, antiperspirants and/or deodorants, shower gels and skin creams, including suntan creams skin creams, skin care lotions, moisturisers, facial treatments such as acne or wrinkle removers, personal and facial cleansers, bath oils, perfumes, fragrances, colognes, sachets, sunscreens, pre-shave and after shave lotions, shaving soaps and shaving lathers, depilatories, or cuticle coats. In such applications the inclusion of the products of the present invention result in enhanced moisturisation, skin feel and improved foam generation in the respective compositions.

For use on the skin, the compositions according to the present invention may be used in a conventional manner for example for conditioning the skin. An effective amount of the composition for the purpose is applied to the skin. Such effective amounts generally range from about 1 mg/cm² to about 3 mg/cm². Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The siloxanes of the invention can in general be used in the textile industry as fibre lubricants, fabric softeners and/or anti-wrinkle agents, and can also be used as ingredients of polishes or protective coatings. Other applications for the products of the present invention include plastic additives, hydraulic fluids, vibration damping, release agents, antifoamers, dielectric media, water repellents, surfactants; greases, coagulants, heat transfer media, polishes and lubricants and the like.

In accordance with a further embodiment of the present invention there is provided the use of siloxane salt compound obtainable by reacting an amino acid derivative selected from the group of an N-acyl amino acid and an N-aroyl amino acid with an amino functional siloxane optionally in the presence of a solvent, in a hair care and/or skin care product.

In accordance with a further embodiment of the present invention there is provided the use of siloxane amide compound obtainable by reacting an amino acid derivative selected from the group of an N-acyl amino acid and an N-aroyl amino acid with an amino functional siloxane in the absence of a solvent, in a hair care and/or skin care product.

In accordance with a further embodiment of the present invention there is provided the use of siloxane amide compound obtainable by reacting an amino acid derivative selected from the group of an N-acyl amino acid and an N-aroyl amino acid with an amino functional siloxane optionally in the absence of a solvent, in the manufacture of a hair care and/or skin care product.

The invention is illustrated by the following Examples

### EXPERIMENTAL

The following were used in all examples:-
Acetyl-amino acids were obtained from Sigma Aldrich or Fisher and were used as received. Ethanol used was a standard denatured lab Grade.
All NMR analysis was carried out using a JEOL Lambda running spectrometer operating at a frequency of at 400MHz for proton analysis. Deuterated Chloroform was the solvent used during nmr analysis. All viscosities provided are at 25°C unless otherwise indicated. Henceforth the term DP is used to mean Degree of Polymerisation.

### EXAMPLE 1 "NON-SOLVENT METHOD"

The amino functional siloxane and the N-Acetyl amino acid (1 molar equivalent per nitrogen of the siloxane) were charged to a round bottom flask fitted with a mechanical stirrer. The flask was then evacuated (reaction can also be run under nitrogen if salt is desired product) and the reaction mixture heated to 120°C over an hour, the mix tends to clear during this stage. If the salt was the desired product the reaction was stopped at this stage and product was decanted off. If the amide was the desired product the mix was further heated to 140-150°C for 4-5 hours. The resulting product was characterised by ¹H NMR. For aminopropyl functional siloxanes there is a characteristic shift of the CH₂N group from 2.6ppm (amine) to 2.7ppm (ammonium salt) to 3.1 ppm (amide).

Both the salt and the amine products of amino functional siloxanes and N-Acetylamino acids could be prepared thermally by mixing the reagents in the absence of solvent and heating under nitrogen or vacuum. In general it was found that the reaction mixture became clear at around 100°C and that this corresponded to formation of the salt. Further heating to around 150°C under vacuum resulted in the mix becoming cloudy and formation of the amide. Reactions were followed by proton NMR in which there was a characteristic shift of the CH₂ group alpha to the nitrogen from 2.6ppm for the amine to 2.75ppm for the salt to 3.1 ppm for the amide. Amide formation was generally slow and 4 to 8 hours were required to drive reaction to over 80% conversion. It was found that selective preparation of the salt could be reasonably well controlled but that the product could contain up to 10% of the amide.

The properties of the materials produced utilising the thermal route are summarised in Table 1. In addition to the products shown in Table 1 the reaction of the 100DP Aminopropyl endblocked siloxane with N-Acetyl-Glutamine (1) was attempted but gave a brown intractable solid, this reaction was not pursued further.

The reactions of aminopropyldimethyl terminated dimethylmethylaminopropyl siloxane (viscosity = 1500 mPa.s) with Aspartic Acid, Glycine anhydride and Valine all failed with no reaction occurring.

**TABLE 1**

| **Siloxane** | **Amino Acid** | **Product (Conversion)** | **Appearance** |
|---|---|---|---|
| aminopropyldimethyl terminated polydimethyl siloxane (DP = 11) | N-Acetyl Glycine (AcGly) | Salt | Hard wax, white |
| | | Amide | Hard wax, brown |
| Aminopropyldimethyl terminated polydimethylsiloxane (DP = 100) | AcGly | Salt | White cloudy semisolid |
| | | Amide | Yellow opaque semisolid |
| | N-Acetyl-Alanine (AcAla) | Salt | Translucent white solid |
| | | Amide | Cream, opaque, high viscosity fluid |
| | N-Acetyl-Valine (AcVal) | Salt | Clear solid (becomes cloudy on prolonged storage) |
| | | Amide | White, opaque, extremely high viscosity fluid |
| | 4-Acetamido-benzoic acid (3) | Salt | White cloudy very high viscosity fluid |
| | 2-Pyrrolidone-5-carboxylic acid (2) | Amide | Cloudy grey high viscosity fluid |
| Aminopropyldimethyl terminated dimethyl methyl aminopropyl siloxane (viscosity = 1500 mPa.s) | AcGly | Salt | Hazy semi-solid |
| trimethyl terminated dimethyl aminoethyl aminoisobutyl siloxane (viscosity =3000 mPa.s) | AcVal | Salt | Slightly hazy colourless solid |

Comparative reactions were attempted with several non-acylated amino acids, namely aspartic acid, valine and glycyl-glycine. As might be expected these were unsuccessful.

### EXAMPLE 2

The amino functional siloxane and the N-acetyl amino acid (1 molar equivalent per nitrogen of the siloxane) and an approximately equal mass of ethanol were charged to a flask equipped with a magnetic stirrer. The reaction mixture was stirred under ambient conditions for 10 minutes or longer until the mix became homogeneous and one phase or until all the N-acetyl amino acid had dissolved. The product was decanted off and ethanol removed by evaporation if desired. The resulting products were diluted by removing 1 ml of the ethanol solution and adding a further 5mls of pure ethanol, the resulting solution was kept for 1 to 2 hours and observed. A few drops of this solution were placed on a glass microscope slide and allowed to dry to determine film forming behaviour. The ethanol solution was further diluted by addition of 5mls of distilled water and the result mixture kept at least overnight.

The above process was used to prepare a series of salts using a selection of alternative reactants as indicated in Table 2 below. It was found that the properties of the salts prepared depended on both the amino acid derivative and on the siloxane.

**TABLE 2**

| **Siloxane** | **N-Acetyl-Glycine** | **N-Acetyl-Alanine** | **N-Acetyl-Valine** |
|---|---|---|---|
| Aminopropyldimethyl terminated polydimethyl siloxane (DP = 11) | Powdery yellow solid | | |
| Aminopropyldimethyl terminated polydimethylsiloxane (DP = 100) | White Cloudy Semi-solid | Translucent White semisolid | Clear, semisolid |
| Aminopropyldimethyl terminated dimethyl methylaminopropyl siloxane (viscosity = 1500 mPa.s) | Cloudy high viscosity fluid | Clear high viscosity fluid | Clear, semisolid |
| trimethyl terminated dimethyl aminoethyl aminoisobutyl siloxane (viscosity =3000 mPa.s) | Clear, Extremely high viscosity fluid | | Clear, Non-sticky rubbery solid |

The appearance and rheological behaviour of the products is dependant on both the amino acid derivative and the amino polymer. Increasing the siloxane DP from 11 to 100 with aminopropyl end blocking results in the products becoming much more liquid like as expected given the much higher siloxane ratio. The products also become much less ethanol and ethanol/water soluble again as expected given the increasing dilution of polar end groups by non-polar polydimethylsiloxane (PDMS) backbone. Comparison of the products derived from the 100 DP aminopropyl endblocked siloxane and from aminopropyldimethyl terminated dimethylmethylaminopropyl siloxane (viscosity = 1500 mPa.s) both of which contain a very similar level of nitrogen shows increased ethanol solubility for the aminopropyldimethyl terminated dimethylmethylaminopropyl siloxane (viscosity = 1500 mPa.s) derived product. Aminopropyldimethyl terminated dimethylmethylaminopropyl siloxane (viscosity = 1500 mPa.s) has an average DP of 300 and will thus have around six amine groups per chain compared to the two amine groups per chain for the aminopropyl end-blocked siloxane. This increased number of polar groups per chain is thought to be responsible for the increased solubility.

A comparison of the amide verses the salt products showed that the amides had reduced solubility in ethanol and have somewhat reduced viscosity. These results are not unexpected given the reduced polarity of the amide group compared to the salt.

In general it was found that for materials produced by both the thermal and the ethanol based routes that the observed properties were very similar as was spectrographic characterisation. For the ethanol solvent route no amide formation is observed and this may account for some minor differences from the thermal route where a few percent of the amide is generally present. The materials prepared by the ethanol route generally display better clarity which may be linked to the absence of amide or better dispersion of the amino acid during synthesis.

Various formulations were prepared for shampoo's, conditioners and skin care creams using two of the preferred salts prepared in the presence of a solvent as described in Example 2 above.

The salts utilised in the following examples were
Salt solution 1 : product of the reaction between trimethyl terminated dimethyl aminoethyl aminoisobutyl siloxane (viscosity =3000 mPa.s) and N-acetyltryptophan in ethanol solution at 50%;
Salt solution 2 =: Aminopropyldimethyl terminated polydimethylsiloxane (DP = 100) + N-acetylglycine, salt in ethanol solution at 50%
Hence when, for example, 4% by weight of salt solution was introduced into the composition in the form of a 50 % solution of solvent hence 2% by weight of the actual salt itself was introduced into the composition.

Standard formulations including opaque and clear shampoos, rinse-off conditioners, pump spray, and Oil in Water (O/W) and Water in Oil (W/O) creams were prepared using the above salts to assess their potential use in hair care and skincare products.

### EXAMPLE 3 RINSE-OFF CONDITIONER

**TABLE 3A**

| | **Ingredients** | **INCI name** | **Control** | **Sample 3.1** | **Sample 3.2** |
|---|---|---|---|---|---|
| *Phase A* | | | | | |
| | Natrosol^{®} 250HHR | Hydroxyethyl cellulose (HEC) | 1.5% | 1.5% | 1.5% |
| | Arquad^{®} 16-29 | Cetrimonium chloride (29%) | 0.3% | 0.3% | 0.3% |
| | Water | | 50.0% | 50.0% | 50.0% |
| *Phase B* | | | | | |
| | Cetyl alcohol | Cetyl alcohol | 1.0% | 1.0% | 1.0%- |
| | Arlacel^{®} 165 | PEG-100 stearate& Glyceryl stearate | 1.0% | 1.0% | 1.0% |
| | Salt solution 1 | | - | 4% | - |
| | Salt solution 2 | | - | - | 4% |
| *Phase C* | | | | | |
| | Water | | up to 100% | up to 100% | up to 100% |
| *Phase D* | | | | | |
| | Citric acid | | q.s. | q.s. | q.s. |
| Viscosity | After 24h (mPa.s) | . | 37 800 | 43 000 | 43 200 |
| Stable for | | | 2 months | 3 weeks | 6 months |

| | | | | | |
|---|---|---|---|---|---|
| (PEG = polyethylene glycol) | | | | | |

### PROCEDURE

Part A in Table 3a was prepared by sifting the HEC (Natrosol^{®} 250HHR from Hercules/Aqualon) into the water while stirring rapidly. After all the HEC was dispersed, the Cetrimonium chloride (Arquad^{®} 1629 from Akzo Nobel Chemicals) was introduced. Stirring was then continued until the HEC went into solution.

The ingredients for Part B are mixed together and heated to 80-85°C. Arfacel^{®} 165 is from Uniqema. The ingredients for Part C are mixed together and heated to 80-85°C. The resulting Part B mixture is then added rapidly to the resulting Part C mixture with rapid stirring and the resulting composition is mixed together for 5-10 minutes. Subsequently Part A (at room temperature) is introduced into the hot part B & C emulsion over a period of 2-3 minutes so as not to cool the emulsion down too quickly. Mixing was continued until resulting mixture had cooled to 40-45° C. Water was introduced to compensate for water loss and the pH was adjusted to 4 with phase D.

A second run was done in order to evaluate the performances of Salt solution 2 in a rinse off application on hair using the compositions in Table 3b below in which Comparative 1 (Comp. 1) comprises Crodasone Cystine as the active ingredient and Comparative 2 (Comp. 2) uses trimethyl terminated dimethyl aminoethyl aminoisobutyl siloxane (viscosity = 3000 mPa.s) (TTDAAS) as the active ingredient.

**TABLE 3B**

| | **Ingredients** | **INCI name** | **Control** | **Comp. 1** | **Sample 3.3** | **Comp .2** |
|---|---|---|---|---|---|---|
| *Phase A* | | | | | | |
| | Natrosol^{®} 250HHR | Hydroxyethyl cellulose | 1.5% | 1.5% | 1.5% | 1.5% |
| | Arquad^{®} 16- 29 | Cetrimonium chloride (29%) | 0.3% | 0.3% | 0.3% | 0.3% |
| | Water | | 50.0% | 50.0% | 50.0% | 50.0% |
| *Phase B* | | | | | | |
| | Cetyl alcohol | Cetyl alcohol | 1.0% | 1.0% | 1.0% | 1.0% |
| | Arlacel° 165 | PEG-100 stearate& Glyceryl stearate | 1.0% | 1.0% | 1.0% | 1.0% |
| | Crodasone Cystine | Aqua (and) Cystine bis-PG- Propyl Silanetriol | - | 2% active = 10% materi al | - | - |
| | Salt solution 2 | | - | - | 4% | - |
| | TTDAAS (viscosity = 3000 mPa.s) | | - | - | - | 2% active |
| *Phase C* | | | | | | |
| | Water | | up to 100% | up to 100% | up to 100% | up to 100% |
| *Phase D* | | | | | | |
| | Citric acid | | q.s. | q.s. | q.s. | q.s. |

Results on wet combing (slightly bleached hair) : trimethyl) terminated dimethyl aminoethyl aminoisobutyl siloxane (viscosity = 3000) gave excellent results when used in the rinse-off conditioner formulation, Sample 3.3 also performed well, more than Crodasone Cystine and the Control.

### EXAMPLE 4 OPAQUE SHAMPOO -

A 2% solution of Carbopol^{®} ETD2020 (from Noveon) in water was initially prepared and once completely dispersed was neutralized with KOH or NaOH (20% solution - up to pH 5). The Texapon^{®} A400 (from Cognis) and Comperlan^{®} KD (from Cognis) were then mixed and heated to about 65°C. Once the Texapon^{®} A400 and Comperlan^{®} KD had melted, heating was discontinued and the 2% solution of Carbopol^{®} ETD2020 in water was introduced followed by the appropriate Salt and the mixture was then allowed to cool down to room temperature (approximately 25°C). The viscosity of the resulting mixture was adjusted as required using NaCl and if required water was added to compensate for water loss during the preparation. The pH of the resulting composition was then adjusted to pH to 5-6. The formulations used are depicted in Table 4 below.

**TABLE 4**

| **Ingredients** | **INCI name** | **Control** | **Sample 4.1** | **Sample 4.2** |
|---|---|---|---|---|
| Texapon^{®} A400 | Ammonium Lauryl Sulfate | 30% | 30% | 30% |
| Comperlan^{®} KD | Cocamide DEA | 3% | 3% | 3% |
| Carbopol^{®} ETD2020 (2% solution) | Acrylates/C10-30 AlkylAcrylate Crosspolymer | 30% | 30% | 30% |
| Salt solution 1 | | - | 6% | - |
| Salt solution 2 | | - | - | 6% |
| NaCl | Sodium chloride | q.s. | q.s. | q.s. |
| Water | Water | up to 100% | up to 100% | up to 100% |
| Viscosity 24h (mPa.s) | | 80 | 16 | 24 |
| Stable for | | 6 months | 6 months | 1 day |

After 24h, shampoo containing salt solution 2 appears to be separated: there is a thin layer on top of shampoo. The shampoo containing Salt solution 1 is clear, while Control is hazy. Despite the very low viscosity, the shampoo containing Salt solution 1 is stable for 6 months.

### EXAMPLE 5 - CLEAR SHAMPOO -

A series of compositions containing a variety of active ingredients and comparative active ingredients were prepared as indicated in Table 5. The ingredients of phase A were mixed together and heated to 60°C. The ingredients of phase B were premixed together and then introduced into phase A and inter-mixed with temperature being maintained at about 60°C. The resulting mixture was then allowed to cool room temperature and the pH was adjusted to 6 using phase D. Comparative 1 (Comp. 1) uses Crodasone Cystine as the active ingredient and Comparative 2 (Comp.2) uses trimethyl terminated dimethyl aminoethyl aminoisobutyl siloxane (viscosity = 3000 mPa.s) (TTDAAS) as the active ingredient. Empicol^{®} ESB3 was obtained from Albright & Wilson, Glucamate^{®} DOE120 was obtained from Noveon. Rewoderm^{®} S1333 was obtained from Degussa. Amonyl^{®} 380BA was obtained from Seppic.

**TABLE 5**

| | **Ingredients** | | **INCI name** | **Control** | **Sample 5.1** | **Comp. 1** | **Comp. 2** |
|---|---|---|---|---|---|---|---|
| *Phase A* | | | | | | | |
| | Empicol^{®} ESB3 | | Sodium Laureth Sulfate | 30.0% | 30.0% | 30.0% | 30.0% |
| | Water | | | up to 100% | up to 100% | up to 100% | up to 100% |
| | Glucamate^{®} DOE120 | | PEG-120 Methyl Glucose Dioleate | 1.5% | 1.5% | 1.5% | 1.5% |
| | Rewoderm^{®} S1333 | | Disodium Ricinoleamido MEA-Sulfosuccinate | 4.0% | 4.0% | 4.0% | 4.0% |
| | Amonyl^{®} 380BA | | Cocamidopropyl Betaine | 4.0% | 4.0% | 4.0% | 4.0% |
| *Phase B* | | | | | | | |
| | Comperlan^{®} KD | | Cocamide DEA | 4.0% | 4.0% | 4.0% | 4.0% |
| | Salt solution 1 | | | - | 4% | - | - |
| | Crodasone Cystine | | Aqua (and) Cystine bis-PG-Propyl Silanetriol | - | - | 2% active = 10% material | - |
| | TTDAAS (VISCOSITY = 3000 mPa.s) | | | - | - | - | 2% |
| *Phase D* | | | | | | | |
| | | Citric acid (50%) to pH=6 | | q.s. | q.s. | q.s. | q.s. |
| *Aspect* | | | | Clear | Not clear | Clear | Clear |

### EXAMPLE 6 -SEMI-PERMANENT COLORING CREAM

In this example a semi-permanent colouring cream was prepared using the following method. Phase A was melted, while stirring, at a temperature of 70°C. The phase B ingredients were mixed together in an alternative container, dissolved completely while stirring and gently heated at 40°C. The phase C ingredients were heated separately to 70°C. Phase B was then added to phase A, followed by the phase C ingredients. The resulting mixture was then stirred until homogeneous at which point phase D was added. The ingredients of phase E were added when temperature had decreased to about 40°C. Once the temperature of the mixture had returned to about room temperature the pH was adjusted to 6 with phase F and water was introduced as compensation for water loss due to heating. The compositions used are Tabulated in Table 6 below.

**TABLE 6**

| | **Ingredients** | **INCI name** | **Control** | **Comp.1** | **Sample 6.1** | **Sample 6.2** |
|---|---|---|---|---|---|---|
| *Phase A* | | | | | | |
| | Base O/W 097 | Ceteareth 25/ PEG-2 Stearate/ Paraffinum liquidum/Hydrogenated coconut oil/Cetyl alcohol/ Sodium Stearate | 28.00% | 28.0% | 28.00% | 28.00% |
| *Phase B* | | | | | | |
| | Solvariane^{®} | Laureth 8/ Cocotrimonium Chloride/Butoxyethanol / PEG-7 Glyceryl cocoate/Quaternium-80 | 9.00% | 9.00% | 9.00% | 9.00% |
| | Propylene glycol | Propylene Glycol | 4.00% | 4.00% | 4.00% | 4.00% |
| | Phenoxetol | Phenoxyethanol | 0.30% | 0.30% | 0.30% | 0.3% |
| | Covariane^{®} Rouge W3123 | HC Red 3 (4-(2'-hydroxyethyl) amino-3-nitroaniline) | 2.00% | 2.00% | 2.00% | 2.00% |
| | Covastyle^{®} TBQ | t-butyl hydroquinone | 0.20% | 0.20% | 0.20% | 0.20% |
| *Phase C* | | | | | | |
| | Water | - | to 100% | to 100% | to 100% | to 100% |
| *Phase D* | | | | | | |
| | EDTA | Disodium EDTA | 0.25% | 0.25% | 0.25% | 0.25% |
| *Phase E* | | | | | | |
| | TTDAAS (Viscosity = 3000 mPa.s) | Amodimethicone | - | 1.40% | - | - |
| | Salt solution 1 | | - | - | 2.80% | - |
| | Salt solution 2 | | - | - | - | 2.80% |
| *Phase* F | | | | | | |
| | AMP10%(*) to pH=6 | Aminomethyl Propanol | q.s. | q.s. | q.s. | q.s. |

Base O/W 097, Solvariane^{®} , Covariane^{®} Rouge W3123, Covastyle^{®} TBQ were obtained from LCW - Sensient Cosmetic Technologies of France

### EXAMPLE 7 PUMP HAIR SPRAY (80% VOC - formulation)

Pump hairspray formulations were prepared using the following method. The phase A ingredients were initially mixed. The phase B ingredients were then added during mixing and the composition was mixed until phase B had dissolved at which point in time, phase C was added and the resulting composition was mixed until homogenous. The formulations used are depicted in Table 7 below

**TABLE 7**

| | **Ingredients** | **INCI name** | **Control** | **Sample 7.1** | **Sample 7.2** |
|---|---|---|---|---|---|
| *Phase A* | | | | | |
| | Ethanol | Ethanol | 80.00% | 78.80% | 78.80% |
| | AminoMethyl Propanol | AminoMethy I Propanol | 1.39% | 1.39% | 1.39% |
| | Water | Water | 12.61% | 12.61% | 12.61% |
| *Phase B* | | | | | |
| | Luvimer^{®} 100P | Acrylates Copolymer | 6.00% | 6.00% | 6.00% |
| *Phase C* | | | | | |
| | Salt solution 1 | | - | 1.2% | - |
| | Salt solution 2 | | - | - | 1.2% |

| | | | | | |
|---|---|---|---|---|---|
| Luvimer^{®} 100P was obtained from BASF Corporation. | | | | | |

### EXAMPLE 8 SHOWER GEL

A series of shower gel formulations were prepared using the following process. Mix phase A ingredients. Add phase B while mixing. Add phase C slowly while mixing. Mix uniformly over the tank. Add phase D. Adjust pH to 6-7. The formulations used are shown in Table 8 below.

**TABLE 8**

| | **Ingredients** | **INCI name** | **Control** | **Sample 8.1** | **Sample 8.2** |
|---|---|---|---|---|---|
| Phase A | | | | | |
| | Empicol^{®} ESB3 | Sodium Laureth Sulfate | 30% | 30% | 30% |
| | Oramix^{®} NS10 | Decyl Glucoside | 5% | 5% | 5% |
| | Amonyl^{®} 380BA | Cocamidopropyl Betaine | 10% | 10% | 10% |
| Phase B | | | | | |
| | Brij^{®} 30 | Laureth-4 | 2% | 2% | 2% |
| | Sepigel^{®} 305 | Polyacrylamide (and) C13-14 Isoparaffin (and) Laureth-7 | 2% | 2% | 2% |
| Phase C | | | | | |
| | Water | | up to 100% | up to 100% | up to 100% |
| Phase D | | | | | |
| | Salt solution 1 | | / | 10% | |
| | Salt solution 2 | | / | / | 10% |
| Phase E | | | | | |
| | KOH (10% solution) | | q.s. | q.s. | q.s. |

| | | | | | |
|---|---|---|---|---|---|
| Empicol^{®} ESB3 was obtained from Albright & Wilson Oramix^{®} NS10, Amonyl^{®} 380BA and Sepigel^{®} 305 were obtained from Seppic. Brij^{®} 30 was obtained from Uniqema. | | | | | |

### EXAMPLE 9 O/W CREAM

Samples of Oil in water based creams were prepared using the following methods. The phase A ingredients were mixed (gently) and heated to 60°C, the mixture was covered in an attempt to prevent evaporation of solvents. Phase B was then introduced into phase A at 60°C followed by phase C and the resulting composition was allowed to cool down to room temperature once phase C had been completely introduced.

All creams prepared using Salt solution 2 were white. The viscosity was seen to increase when the cyclomethicone and dimethicone cross polymer level is up to 10%, but stability is not necessarily improved. Most stable cream containing Salt solution 2 is composition 2a, containing only 5% of cyclomethicone and dimethicone cross polymer and 10% cyclomethicone. The best cream containing Salt solution 1 is 1 (b), with 5% cyclomethicone and dimethicone cross polymer and 15% cyclomethicone. It requires longer mixing after all ingredients are added to achieve a nice emulsion (15 minutes). The formulations prepared are depicted in Tables 9A and 9B below.
Myritol^{®} 312 was obtained from Cognis. Phenonip® was obtained from Clariant Corporation. Emulium Delta was obtained from Gattefosse s.a.s. of France. Keltrol^{®} was obtained from CP Kelco.

**TABLE 9A**

| **Ingredients** | | **INCI name** | **Comp.1** | **Comp.2** | **Sample 9.1** |
|---|---|---|---|---|---|
| *Phase A* | | | | | |
| | | Cyclomethicone | 15.0% | 15.0% | 10.0% |
| | | Cyclomethicone/ Dimethicone Crosspolymer | 10.0% | 10.0% | 5.0% |
| | Jojoba oil | Simmondsia Chinensis (Jojoba) Seed Oil | 2.0% | 2.0% | 2.0% |
| | Myritol^{®} 312 | Caprilic/Capric Triglyceride | 2.0% | 2.0% | 2.0% |
| | Phenonip® | Phenoxyethanol/ Methylparaben/ Ethylparaben/ Propylparaben/ Butylparaben | 0.5% | 0.5% | 0.5% |
| | Salt solution 2 | | - | - | 10.0% |
| *Phase B* | | | | | |
| | Emulium Delta | Cetyl alcohol/ Glyceryl stearate / PEG_75Stearate/ Ceteth-20/ Steareth-20 | 4.0% | 4.0% | 4.0% |
| *Phase C* | | | | | |
| | Water | | up to 100% | up to 100% | up to 100% |
| | Glycerin | Glycerin | 2% | 2% | 2% |
| | Keltrol^{®} | Xanthan Gum | 0.20% | 0.20% | 0.20% |
| Viscosity 24 h (mPa.s) | | | 14 400 | 15 200 | 25 000 |
| Stable for | | | 6 months | 6 months | 6 months |

### O/W CREAM

**TABLE 9B**

| | **Ingredients** | **INCI name** | **Sample 9.2** | **Sample 9.3** | **Sample 9.4** |
|---|---|---|---|---|---|
| *Phase A* | | | | | |
| | | Cyclomethicone | 10.0% | 15.0% | 15.0% |
| | | Cyclomethicone/ Dimethicone Crosspolymer | 10.0% | 5.0% | 5.0% |
| | Jojoba oil | Simmondsia Chinensis (Jojoba) Seed Oil | 2.0% | 2.0% | 2.0% |
| | Myritol^{®} 312 | Caprilic/Capric Triglyceride | 2.0% | 2.0% | 2.0% |
| | Phenonip® | Phenoxyethanol Methylparaben/ Ethylparaben/ Propylparaben/ Butylparaben | 0.5% | 0.5% | 0.5% |
| | Salt solution 1 | | - | - | 10.0% |
| | Salt solution 2 | | 10.0% | 10.0% | - |
| *Phase B* | | | | | |
| | Emulium Delta | Cetyl alcohol/ Glyceryl stearate/ PEG_75 Stearate/ Ceteth-20/ Steareth-20 | 4.0% | 4.0% | 4.0% |
| *Phase C* | | | | | |
| | Water | | up to 100% | up to 100% | up to 100% |
| | Glycerin | Glycerin | 2% | 2% | 2% |
| | Keltrol^{®} | Xanthan Gum | 0.20% | 0.20% | 0.20% |
| Viscosity 24 h (mPa.s) | | | 45 000 | 24 800 | 52 000 |
| Stable for | | | 2 months | 1 month | 5 months |

A triangular sensory test was run to compare the creams containing salts in accordance with the present invention versus the control gave following results:
- Salt solution 1 cream feels more draggy, more tacky, is less easy to rub in and less watery, all indicating a richer cream (difference at 99,9% confidence level)
- Salt solution 2 cream leaves more film on skin - all other parameters were not well defined - cream less stable (difference at 99,9% confidence level).

### EXAMPLE 10 W/O CREAM

Formulations of water in oil cream were prepared using the following method. The phase A ingredients were mixed in a foamer mixer. The phase B ingredients were mixed together and phase B was then slowly added to phase A (using e.g. a dropping funnel) and the resulting mixture was stirred for 15 minutes after the completion of the addition of phase B and then was transported through a high shear mixer. The formulations prepared are depicted in Table 10 below.

**Table 10**

| | **Ingredients** | **INCI name** | **Control** | **Sample 10.1** | **Sample 10.2** |
|---|---|---|---|---|---|
| *Phase A* | | | | | |
| | Mineral oil | Mineral oil | 20.0% | 20.0% | 20.0% |
| | Lauryl PEG/PPG-18/18 Methicone | Lauryl PEG/PPG-18/18 Methicone | 2.0% | 2.0% | 2.0% |
| | Salt solution 1 | | - | 5.0% | - |
| | Salt solution 2 | | - | - | 5.0% |
| *Phase B* | | | | | |
| | Water | Water | up to 100% | up to 100% | up to 100% |
| | NaCl | Sodium Chloride | 1.0% | 1.0% | 1.0% |
| | Viscosity 24h (mPa.s) | | 45600 | 89600 | 99200 |
| | Stable for | | 6 months | 6 months | 6 months |

The mineral oil used was Klearol^{®} a pure white mineral oil sold by the United States Oil Company and having a kinematic viscosity of about 8.5mm²/s at 40°C when measured in accordance with ASTM D 445.

Formulated creams are of a good consistency and rich.

## Claims

1. A method of preparing an amino acid functional siloxane by reacting an amino acid derivative selected from the group of an N-acyl amino acid and an N-aroyl amino acid with an amino functional siloxane optionally in the presence of a solvent.

2. A method in accordance with claim 1 wherein the N-acyl group is selected from N-acetylated, N-propanoyl, N-butanoyl, N-pentanoyl and N-hexanoyl groups.

3. A method in accordance with any preceding claim wherein the N-acyl amino acid is an N-acetylated amino acid selected from the group of N-Acetyl-Glycine, N-Acetyl-Alanine N-acetyltryptofan, N-Acetyl-Valine N-Acetyl-Glutamine or 2-Pyrrolidone-5-Carboxylic Acid and 4-Acetamido-benzoic acid.

4. A method in accordance with any preceding claim wherein the aminosiloxane includes siloxane units of formula (4)
-(RₐSiO_{(4-a)/2})- (4)
in which each R is independently an organic group such as an aliphatic carbon group having from 1 to 10 carbon atoms optionally substituted with one or more amine, alkoxy, hydroxy, or fluorine and a is 0, 1 or 2.

5. A method in accordance with any preceding claim wherein substantially all of the groups R are alkyl or aminoalkyl groups.

6. A method in accordance with claim 5 wherein each R group may be the same or different and are selected from the group of methyl, ethyl, propyl, butyl, vinyl, cyclohexyl, phenyl, tolyl group, aminomethyl, aminoethyl, aminopropyl, aminobutyl, aminoisobutyl, aminocyclohexyl, aminophenyl or aminotolyl group, or an aminoethylaminoisobutyl group or an aminoethylaminopropyl group, or 3,3,3-trifluoropropyl or a beta-(perfluorobutyl)ethyl group.

7. A method in accordance with any preceding claim **characterised** that in the absence of a solvent the reaction is carried out at a temperature above room temperature (25°C) but below the boiling/decomposition point of the amino acid.

8. A method in accordance with claim 7 wherein the reaction temperature is between 75°C and 150°C.

9. A method in accordance with any preceding claim wherein in the absence of a solvent, the reaction is carried out in an inert atmosphere (e.g. nitrogen) or under vacuum.

10. A method in accordance with any preceding claim wherein the solvent is an alcohol such as ethanol, propanol, isopropanol, butanol, pentanol and hexanol or mixtures thereof.

11. A method in accordance with any preceding claim wherein in the presence of a solvent the reaction is carried at a temperature between room temperature and 50°C.

12. A siloxane salt compound obtainable by the method in accordance with any preceding claim.

13. An amide compound obtainable by the method in accordance with claim 1 in the absence of a solvent.

14. Use of a siloxane salt in accordance with claim 12 in an ointment, cream, gel, paste, foam aerosol.

15. Use of an amide in accordance with claim 13 in an ointment, cream, gel, paste, foam aerosol.

16. Use of a compound in accordance with claim 12 or 13 in a hair care product or a skin care product.

17. A personal care product comprising one or more compounds in accordance with claim 12 or 13 and an acceptable carrier material.

18. A personal care product in accordance with claim 17 comprising a compound in accordance with claims 12 or 13 in either a water in oil emulsion or an oil in water emulsion composition.

19. A personal care product in accordance with claim 17 or 18 comprising an oil (nonaqueous) phase, and an aqueous phase in one or both of which an emulsifier is present.

## Patentansprüche

1. Ein Verfahren zum Herstellen eines aminosäurefunktionellen Siloxans durch In-Reaktion-Bringen eines Aminosäurederivats, ausgewählt aus der Gruppe einer N-Acylaminosäure und einer N-Aroylaminosäure mit einem aminofunktionellen Siloxan, gegebenenfalls in Gegenwart eines Lösungsmittels.

2. Ein Verfahren gemäß Anspruch 1, wobei die N-Acylgruppe ausgewählt ist aus N-acetylierten Gruppen, N-Propanoyl-, N-Butanoyl-, N-Pentanoyl und N-Hexanoylgruppen.

3. Ein Verfahren gemäß einem der vorhergehenden Ansprüche, wobei die N-Acylaminosäure eine N-acetylierte Aminosäure ist, ausgewählt aus der Gruppe aus N-Acetylglycin, N-Acetylalanin, N-Acetyltryptophan, N-Acetylvalin, N-Acetylglutamin oder 2-Pyrrolidon-5-Carbonsäure und 4-Acetamidobenzoesäure.

4. Ein Verfahren gemäß einem der vorhergehenden Ansprüche, wobei das Aminosiloxan Siloxaneinheiten der Formel (4)
-(RₐSiO_{(4-a)/2})- (4)
umfasst, wobei jedes R unabhängig voneinander eine organische Gruppe ist, wie etwa eine aliphatische Kohlenwasserstoffgruppe mit 1 bis 10 Kohlenstoffatomen, optional substituiert mit einem oder mehreren von Amin, Alkoxy, Hydroxy, oder Fluor und a gleich 0,1 oder 2 ist.

5. Ein Verfahren gemäß einem der vorhergehenden Ansprüche, wobei im Wesentlichen alle Gruppen R Alkyl- oder Aminoalkylgruppen sind.

6. Ein Verfahren gemäß Anspruch 5, wobei jede R-Gruppe die gleiche oder unterschiedlich sein kann und ausgewählt ist aus der Gruppe aus Methyl-, Ethyl-, Propyl-, Butyl-, Vinyl-, Cyclohexyl-, Phenyl-, Tolylgruppe, Aminomethyl-Aminoethyl-, Aminopropyl-, Aminobutyl-, Aminoisobutyl-, Aminocyclohexyl-, Aminophenyl- oder Aminotolylgruppe oder einer Aminoethylaminoisobutylgruppe oder einer Aminoethylaminopropylgruppe oder 3,3,3-Trifluorpropyl- oder eine β-(Perfluorbutyl)ethylgruppe.

7. Ein Verfahren gemäß einem der vorhergehenden Ansprüche, **dadurch gekennzeichnet, dass** in Abwesenheit eines Lösungsmittels die Reaktion bei einer Temperatur oberhalb von Raumtemperatur (25°C) jedoch unterhalb des Siede-/Zersetzungspunktes der Aminosäure durchgeführt wird.

8. Ein Verfahren gemäß Anspruch 7, wobei die Reaktionstemperatur zwischen 75°C and 150°C liegt.

9. Ein Verfahren gemäß einem der vorhergehenden Ansprüche, wobei in Abwesenheit eines Lösungsmittels die Reaktion in einer inerten Atmosphäre (z. B. Stickstoff) oder unter Vakuum durchgeführt wird.

10. Ein Verfahren gemäß einem der vorhergehenden Ansprüche, wobei das Lösungsmittel ein Alkohol, wie etwa Ethanol, Propanol, Isopropanol, Butanol, Pentanol und Hexanol oder Mischungen davon, ist.

11. Ein Verfahren gemäß einem der vorhergehenden Ansprüche, wobei in Gegenwart eines Lösungsmittels die Reaktion bei einer Temperatur zwischen Raumtemperatur und 50°C durchgeführt wird.

12. Eine Siloxansalzverbindung, erhältlich durch das Verfahren gemäß einem der vorhergehenden Ansprüche.

13. Eine Amidverbindung, erhältlich durch das Verfahren gemäß Anspruch 1 in Abwesenheit eines Lösungsmittels.

14. Verwendung eines Siloxansalzes gemäß Anspruch 12 in einer Salbe, einer Creme, einem Gel, einer Paste, einem Schaumaerosol.

15. Verwendung eines Amids gemäß Anspruch 13 in einer Salbe, einer Creme, einem Gel, einer Paste, einem Schaumaerosol.

16. Verwendung einer Verbindung gemäß Anspruch 12 oder 13 in einem Haarpflegeprodukt oder einem Hautpflegeprodukt.

17. Ein Körperpflegeprodukt, enthaltend eine oder mehrere Verbindungen gemäß Anspruch 12 oder 13 und ein annehmbares Trägermaterial.

18. Ein Körperpflegeprodukt gemäß Anspruch 17, enthaltend eine Verbindung gemäß Anspruch 12 oder 13 entweder in einer in Wasser-in-Öl-Emulsions-oder in einer Öl-in-Wasser-Emulsions-Zusammensetzung.

19. Ein Körperpflegeprodukt gemäß Anspruch 17 oder 18, enthaltend eine Ölphase (nichtwässrige Phase) und eine wässrige Phase, wobei in einer oder in beiden ein Emulgator vorliegt.

## Revendications

1. Procédé de préparation d'un siloxane aminoacide-fonctionnel par réaction d'un dérivé d'aminoacide choisi dans le groupe d'un N-acyl-aminoacide et d'un N-aroylaminoacide avec un siloxane aminofonctionnel, éventuellement en présence d'un solvant.

2. Procédé selon la revendication 1 où le groupe N-acyle est choisi parmi les groupes N-acétylés, N-propanoyle, N-butanoyle, N-pentanoyle et N-hexanoyle.

3. Procédé selon l'une quelconque des revendications précédentes où le N-acyl-aminoacide est un aminoacide N-acétylé choisi dans le groupe de la N-acétylglycine, de la N-acétylalanine, du N-acétyltryptophane, de la N-acétylvaline, de la N-acétylglutamine ou de l'acide 2-pyrrofidone-5-carboxylique et de l'acide 4-acétamidobenzoïque.

4. Procédé selon l'une quelconque des revendications précédentes où l'aminosiloxane inclut des unités siloxane de formule (4)
-(RₐSiO_{(4-a)/2})- (4)
où chaque R est indépendamment un groupe organique comme un groupe carboné aliphatique ayant de 1 à 10 atomes de carbone éventuellement substitué avec un ou plusieurs amines, alcoxy, hydroxy ou fluor et a est 0, 1 ou 2.

5. Procédé selon l'une quelconque des revendications précédentes où sensiblement tous les groupes R sont des groupes alkyle ou aminoalkyle.

6. Procédé selon la revendication 5 où chaque groupe R peut être identique ou différent et sont choisis dans le groupe d'un groupe méthyle, éthyle, propyle, butyle, vinyle, cyclohexyle, phényle, tolyle, d'un groupe aminométhyle, aminoéthyle, aminopropyle, aminobutyle, aminoisobutyle, aminocyclohexyle, aminophényle ou aminotolyle ou d'un groupe aminoéthylaminoisobutyle ou d'un groupe aminoéthyl-aminopropyle, ou d'un groupe 3,3,3-trifluoropropyle ou bêta-(perfluorobutyl)éthyle.

7. Procédé selon l'une quelconque des revendications précédentes **caractérisé en ce que**, en l'absence d'un solvant, la réaction est conduite à une température supérieure à la température ambiante (25°C) mais inférieure au point d'ébullition/décomposition de l'aminoacide.

8. Procédé selon la revendication 7 où la température de réaction est entre 75°C et 150°C.

9. Procédé selon l'une quelconque des revendications précédentes où, en l'absence d'un solvant, la réaction est conduite dans une atmosphère inerte (par exemple l'azote) ou sous vide.

10. Procédé selon l'une quelconque des revendications précédentes où le solvant est un alcool comme l'éthanol, le propanol, l'isopropanol, le butanol, le pentanol et l'hexanol ou des mélanges de ceux-ci.

11. Procédé selon l'une quelconque des revendications précédentes où, en présence d'un solvant, la réaction est conduite à une température entre la température ambiante et 50°C.

12. Composé sel de siloxane pouvant être obtenu par le procédé selon l'une quelconque des revendications précédentes.

13. Composé amide pouvant être obtenu par le procédé selon la revendication 1 en l'absence d'un solvant.

14. Utilisation d'un sel de siloxane selon la revendication 12 dans une pommade, une crème, un gel, une pâte ou un aérosol de mousse.

15. Utilisation d'un amide selon la revendication 13 dans une pommade, une crème, un gel, une pâte, un aérosol de mousse.

16. Utilisation d'un composé selon la revendication 12 ou 13 dans un produit pour les soins des cheveux ou un produit pour les soins de la peau.

17. Produit pour les soins personnels comprenant un ou plusieurs composés selon la revendication 12 ou 13 et un vecteur acceptable.

18. Produit pour les soins personnels selon la revendication 17 comprenant un composé selon la revendication 12 ou 13 dans une composition de type émulsion eau-dans-huile ou émulsion huile-dans-eau.

19. Produit pour les soins personnels selon la revendication 17 ou 18 comprenant une phase huileuse (non aqueuse) et une phase aqueuse dans l'une desquelles ou dans les deux desquelles un émulsifiant est présent.
